# EUROPÄISCHE PATENTANMELDUNG

(11) **EP 1 186 656 A1**
(43) Veröffentlichungstag der Anmeldung: **13.03.2002**
(21) Anmeldenummer: 01119884.3
(22) Anmeldetag: 17.08.2001
(51) Int. Cl.: C12N 7/02, G01N 33/569, A61K 39/12

(54) **Verfahren zur Vermehrung oder zur Entfernung von Circoviren aus biologischem Material**

(30) Priorität: 08.09.2000 DE 10044648
(71) Anmelder: Aventis Behring GmbH, 35002 Marburg (DE)
(72) Erfinder: Bernhardt, Dieter, Dr., 35091 Coelbe (DE); Weimer, Thomas, Dr., 35075 Gladenbach (DE); Groener, Albrecht, Dr., 35041 Marburg (DE)

(57) **Zusammenfassung**

Es werden Verfahren zur Vermehrung und zur Neutralisierung oder Entfernung von Circoviren, insbesondere porcinen Circoviren beschrieben, die aus einer infizierten Zellkultur nach einmaliger oder mehrmaliger Passage in Zellkulturen des Schweines, des Rindes oder des Menschen gewonnen werden. Bei der Vermehrung der porcinen Circoviren tritt in der Zellkultur ein cytopathogener Effekt auf. Die Circoviren lassen sich durch Behandlung mit einem antikörperhaltigen Substrat wie Schweineserum oder humanem Immunglobulin neutralisieren oder durch ein Pasteurisierungsverfahren entfernen. Es werden außerdem eine Vakzine und ein Diagnostikum beschrieben, die inaktivierte oder avirulente Circoviren enthalten.

## Beschreibung

Gegenstand der Erfindung sind Verfahren zur Vermehrung und Quantifizierung der infektiösen bzw. Antigen-Menge und Bestimmung der Antikörper gegen Circoviren, insbesondere porcinen Circoviren (PCV). Außerdem sind Verfahren zum Nachweis der Reduktion von Circoviren und circovirus-ähnlichen Viren des Menschen durch das Herstellungsverfahren von Arzneimitteln aus biologischem Material Gegenstand der Erfindung (1).

Porcine Circoviren sind isometrische, etwa 17 nm große, nicht umhüllte Viren mit einzelsträngiger, circulärer DNS von 1,76 kb. Antikörper gegen das porcine Circovirus sind im Serum von Menschen, Mäusen und Rindern mittels eines indirekten Immunofluoreszenznachweises (IFA) und mittels der ELISA-Methode gefunden worden (2). In Schlachtschweinen gelingt der Antikörpernachweis in 53 bis 92% der Tiere (3). Die klinische Bedeutung von PCV ist bisher unbekannt. Zwischenzeitlich wurde ein neuer Typ des porcinen Circovirus nachgewiesen, der zu Läsionen in Geweben von Schweinen führt und mit Krankheitssymptomen korrelierbar ist; dieser sog. Typ 2 (PCV2) unterscheidet sich vom zuvor genannten Typ 1 (PCV1) hauptsächlich hinsichtlich der Pathogenität (3 und 4).

Obwohl das porcine Circovirus als kontaminierendes Agens in Gewebekulturen vom Schwein nachzuweisen ist, ist es bisher nicht gelungen, das Virus in vitro zu vermehren und einen routinemäßigen Test zur Vermehrung und zum quantitativen Nachweis des Virus, z.B. auf der Basis eines cytopathogenen Effekts (CPE) in den Virus replizierenden Zellen zu etablieren (2).

In vivo allerdings gelingt der Nachweis von PCV in Lymphknoten, Milz-, Tonsillen-, Leber-, Herz-, Lungen-, Nasenschleimhaut-, Nieren-, Bauchspeicheldrüsen- und Darmzellen mittels der Polymerasekettenreaktion (3). Da Schweineorgane zur Transplantation beim Menschen angewendet werden (5), sollte das porcine Circovirus als potientielles Risikovirus für den Menschen betrachtet werden.

Die Aufgabe der vorliegenden Erfindung bestand deshalb darin, eine in vitro Züchtungsmethode für das porcine Circovirus zu entwickeln, um seine Infektiosität nachprüfen zu können. Außerdem sollte ein Verfahren zur Neutralisierung von porcinem Circovirus durch spezifische Antikörper und seine Entfernung aus biologischen Materialien vom Schwein, Menschen oder anderen Wirbeltieren entwickelt werden, damit diese Materialien ohne Bedenken direkt oder indirekt für therapeutische Zwecke, z.B. für die Gewinnung von Insulin, Heparin, Blut und Plasma, Zellkulturmedien und dessen Bestandteilen einschließlich Trypsin, sowie für Zellen zur Produktion rekombinanter Proteine eingesetzt werden können. Schließlich sollte eine erfolgreiche Vermehrung von PCV in Zellkulturen auch die Herstellung einer Vakzine nach an sich bekannten Methoden ermöglichen. Hierbei kann inaktiviertes PCV oder ein avirulenter PCV-Stamm (z.B. durch Selektion eines avirulenten PCV-Stammes nach Adaptation an unterschiedliche Zellkulturen und/oder nach Behandlung infizierter Zellkulturen mit Mutagenen bzw. nach gentechnischer Modifikation des PCV), dann als Lebendimpfstoff eingesetzt werden. Außerdem kann das aus vermehrten porcinen Circoviren gewonnene Antigenmaterial auch für diagnostische Zwecke eingesetzt werden.

Gelöst wird diese Aufgabe durch ein Verfahren zur Vermehrung von Circoviren, insbesondere porcinen Circoviren (PCV), bei dem sich aus einer infizierten Zellkultur gewonnene Circoviren nach einmaliger oder mehrmaliger Passage in Zellkulturen des Schweines, des Rindes oder des Menschen cytopathogenem Effekt vermehren.

Für das erfindungsgemäße Verfahren wurden porcine Circoviren, die aus einer inapparent mit PCV infizierten PK15-Zellkultur (=porcine kidney) gewonnen wurden, nach Passage auf anderen Zellkulturen, insbesondere Schweinezellkulturen, in diesen Zellkulturen unter Entwicklung eines cytopathogenen Effekts vermehrt. Das Vorliegen von porcinen Circoviren wurde dabei mit Hilfe einer spezifischen, ineinandergesetzten ("nested") Polymerasekettenreaktion (PCR) gesichert. Hierfür wurden Primer mit folgenden DNA-Sequenzen eingesetzt:

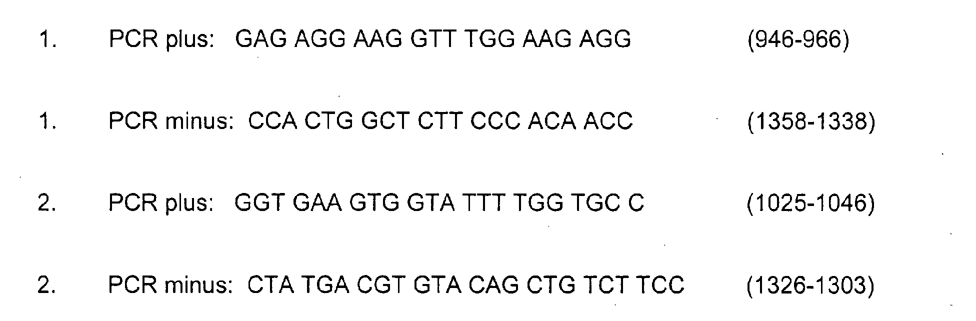

Diese Primer wurden aus dem Origin of Replication ausgewählt (7).

Bei der Durchführung des erfindungsgemäßen Verfahrens wurde beobachtet, dass sich die porcinen Circoviren nicht in allen Zellkulturen verschiedener Säugetiere und des Menschen gleich gut vermehren lassen. Erfolgreich war die Vermehrung in Zellkulturen aus verschiedenen Schweineorganen, aus Rinderniere, Rinderlunge und humaner Lunge. Eine aus fetalem Schweinehoden entwickelte, für die erfolgreiche Vermehrung von porcinem Circovirus gut geeignete Zellkultur ist bei der DSMZ unter der Nr. FSHO-DSM ACC2466 hinterlegt.

Zum quantitativen Nachweis der im Serum vorhandenen Antikörper gegen das porcine Circovirus ist das ELISA-Verfahren gut geeignet. Dabei werden Circoviren nach Adsorption an ein Trägermaterial mit dem zu untersuchenden Serum inkubiert und dadurch ein im Serum enthaltener primärer Antikörper gebunden. Anschließend wird ein gegen den primären Antikörper gerichteter sekundärer, markierter Antikörper damit in Kontakt gebracht und nach Auswaschen des nicht gebundenen sekundären Antikörpers das vom gebundenen markierten Antikörper ausgesendete Lichtsignal (Extinktion) gemessen. Zum Nachweis des Circovirus-Antigens ist die dem Fachmann bekannte "sandwich-Methode" geeignet, wobei ein an ein Trägermaterial gebundener Antikörper gegen Circoviren das Antigen in dem zu untersuchenden Serum bindet; anschließend wird ein gegen Circovirus-Antigen gerichteter (markierter) Antikörper damit in Kontakt gebracht und nach Auswaschen des nicht gebundenen Antikörpers das vom gebundenen markierten Antikörper ausgesendete Lichtsignal (Extinktion) gemessen.

Zur Neutralisierung von Circoviren in biologischem Material sind Seren geeignet, die neutralisierende Antikörper gegen porcines Circovirus enthalten. Neutralisierende Antikörper wurden in Schweineseren und humanem Immunglobulin (γ-Globulin) gefunden. Besonders geeignet zur Neutralisation von Circoviren in biologischem Material ist ein humanes Immunglobulin, das von hochtitrigen humanen Spendern gewonnen wird, die einen mindestens zwei- bis dreifach höheren Titer an spezifischen Antikörpern als Normalspender aufweisen, wobei der durchschnittliche PCV-Antikörpertiter aus einem Plasmapool von mindestens 1.000 Spendern bestimmt wird.

Die Virussicherheit von Arzneimitteln biologischen Ursprungs (z.B. aus Blut/Plasma des Menschen, aus Zellkullturen, aus Organen/Geweben von Tieren) wird entsprechend behördlicher Auflagen untersucht (z.B. CPMP/BWP/268/95: Note for guidance on virus validation studies: the design, contribution and interpretation of studies validating the inactivation and removal of viruses; CPMP/BWP/269/95 rev. 3: Note for guidance on plasma-derived medicinal products); in diesen sogenannten Virusvalidierungsuntersuchungen werden Viren gezielt zu Material verschiedener Produktionsschritte des Herstellungsverfahrens von Biologika zugesetzt und die Entfernung und/oder Inaktivierung der Viren durch den Verfahrensschritt bestimmt. Die für diese Untersuchung benutzten Viren sollten entweder im Ausgangsmaterial der Biologika vorkommen können oder sind, falls diese Viren nicht *in vitro* vermehrbar sind, Modellviren mit möglichst ähnlichen physico-chemischen Eigenschaften wie die kontaminierenden Viren. Ein Modellvirus für das humane Circovirus TTV ist z.B. das PCV. In Untersuchungen eines Verfahrensschrittes bei der Herstellung von Therapeutika aus biologischem Material - der Hitzebehandlung in stabilisierter wässriger Lösung bei 60°C - zeigte sich, daß das porcine Circovirus labil ist und innerhalb weniger Stunden inaktiviert werden konnte; somit läßt sich die Kapazität des Herstellungsverfahrens von Biologika, TTV durch eine Hitzebehandlung zu inaktivieren, belegen. Analog können weitere Verfahrensschritte des Herstellungsprozesses hinsichtlich der Fähigkeit TTV zu entfernen (z.B. durch Präzipitations-, Adsorptions- oder Chromatographie- oder Filtrationsschritte) oder zu inaktivieren (z.B. durch chaotrope Salze oder Substanzen, die in Nukleinsäuren interkalieren, oder durch Bestrahlung mit hochenergetischen Strahlen) mit PCV untersucht werden. Daneben läßt sich die Kapazität, Viren zu inaktivieren und/oder zu entfernen auch z.B. für Zusatzstoffe bei der Produktion von Arzneimitteln ermitteln wie z.B. Seren und andere Medienbestandteile für Zellkulturen zur Produktion rekombinanter Proteine oder monoklonale Antikörper für eine Affinitätschromatographie zur Reinigung und Konzentrierung von Wirkstoffen.

Die Erfindung wird durch die nachfolgenden Beispiele näher erläutert:

### Beispiel 1:

Auf frisch ausgesäte Zellkulturen verschiedener Zelllinien in T25-Zellkulturflaschen wurde der Kulturüberstand einer über viele Gewebepassagen gehaltenen PK15-Kultur, die fünf Tage nach Passagierung in der PCR ein positives Signal für PCV zeigte, im Verhältnis 1:100 abgeimpft.

Dabei wurden folgende permanente Zellkulturen vom Schwein inokuliert:

| | |
|---|---|
| Fetale Schweineniere | = FSN |
| Fetale Schweineschilddrüse | = FSdd |
| Fetale Schweinehoden | = FSHo |
| Fetale Schweinemilz | = FSMi |
| Fetales Schweineherz | = FSHerz |
| Fetale Schweinehaut | = FSHaut |
| Schweineniere | = PS |

Zehn Tage nach Inokulation zeigten alle Kulturen mit Ausnahme der FSdd und FSHaut cytopathogene Veränderungen (CPE). Aus den CPE-positiven Kulturen wurde der Zellkulturüberstand geerntet und bis zur Verwendung für weitere Experimente bei -80°C gelagert.

Auf den homologen Zellkulturen wurde eine zweite Passage des CPE-verursachenden Agens durch Inokulation dieser Zellkulturen mit dem Zellkulturüberstand aus der ersten Passage durchgeführt.

In den PS-Zellen zeigte sich bereits nach vier Tagen ein deutlicher CPE; bei den anderen Kulturen war der CPE erst sechs Tage nach Inokulation sichtbar. Mittels einer spezifischen PCR, bei der die oben genannten Primer eingesetzt wurden, konnte in der PS-Zellkultur, die einen CPE aufwies, PCV nachgewiesen werden, während in den entsprechenden Kontrollzellen ohne Inokulation des PK15-Zellkulturüberstandes kein PCR-Signal zu erkennen war.

### Beispiel 2:

Das in der PS-Zellkultur vermehrte PCV wurde in dem geernteten, niedertourig abzentrifugierten Zellkulturüberstand mittels der Endpunktverdünnungsmethode quantifiziert. Der in 10er-Verdünnungsschritten verdünnte Zellkulturüberstand wurde auf PS-Zellkulturen in Mikrotiterplatten übertragen und die PCV-Vermehrung als cytopathogener Effekt (CPE) ausgewertet. Die Endablesung des Testes erfolgte sieben Tage nach der Infektion und die Berechnung des Virustiters (CCID₅₀ - Cell Culture Infective Dose 50% - in log₁₀) nach dem Fachmann bekannten Methoden (6). Da Circoviren nicht-umhüllte Viren sind, wurde zur Kontrolle und Bestätigung, dass der cytopathogene Effekt auf die Vermehrung von PCV in der Zellkultur zurückzuführen ist, ein Teil des PS-Zellkulturüberstandes mit Chloroform behandelt; diese dem Fachmann bekannte Methode inaktiviert umhüllte Viren. Die vergleichende Titration der nicht-behandelten und der Chloroform-behandelten Virussuspension in PS-Zellen ergab keinen Titerunterschied (Tabelle 1). Dieses Ergebnis, zusammen mit dem spezifischen PCR-Ergebnis, belegt die Vermehrung von PCV in der Zellkultur, insbesondere in Schweine-Zellen.

**Tabelle 1:**

| **Quantitative Bestimmung von in PS-Zellen vermehrten PCV** | |
|---|---|
| **Virussuspension** | **log**_{**10**}**CCID50/ml** |
| PS-Zellkulturüberstand | 7,8 |
| PS-Zellkulturüberstand, chloroform-behandelt | 7,6 |

### Beispiel 3:

Ausgehend von der Virussuspension der PS-Zellkultur - wie in Beispiel 2 beschrieben - wurden frisch ausgesäte Kulturen verschiedener Zellen in T25-Zellkulturflaschen mit einer 1:100 Verdünnung des infektiösen Zellkulturüberstandes geimpft.

Insgesamt wurden drei Adaptierungspassagen durchgeführt; d.h. der mit PCV infizierte Kulturüberstand wurde 1:100 v/v auf frisch angelegte Kulturen der homologen Zellkulturen zehn Tage post infectionem verbracht. Die Kulturen mit cytopathogenem Effekt wurden in der PCR getestet, um die Identität von PCV nachzuweisen. Dabei zeigte sich überraschenderweise, dass sich PCV neben den in Beispiel 1 gezeigten Schweinezellkulturen auch in bestimmten Zellen vom Rind und Menschen unter Ausbildung eines cytopathogenen Effekts vermehrt (Tabelle 2).

**Tabelle 2:**

| **Nachweis der Vermehrung von PCV in Zellkulturen verschiedener Säugetiere und des Menschen** | | | |
|---|---|---|---|
| | **1. Pas- sage*** | **2. Pas- sage*** | **3. Pas- sage*** |
| CRFK = Crandell Feline Kidney | --- | --- | --- |
| KFZI = Feline Kidney | --- | --- | --- |
| MDBK = Madin Darby Bovine Kidney | --- | --- | --- |
| BK Cl10 = Bovine Kidney, Klon 10 | --- | ? | + |
| BK = Bovine Kidney | --- | --- | --- |
| FRLu45 = fetale Rinderlunge | --- | ? | + |
| FROv = fetale Rinderovar | --- | --- | --- |
| FRMi = fetale Rindermilz | --- | --- | --- |
| FC-01 Ni = fetale Cynomolgenniere | --- | --- | --- |
| FRhK 4 = fetale Rhesusniere | --- | --- | --- |
| PH-2 = fetale Cynomolgenniere | --- | --- | --- |
| A549 = Humane Lunge | --- | --- | --- |
| Ma23 = Humane Lunge | --- | --- | --- |
| Mabt = Humane Lunge | ? | + | + |

| | | | |
|---|---|---|---|
| * Erscheinen des CPE --- kein CPE ? CPE fraglich/nicht ausgeprägt * CPE ausgeprägt | | | |

### Beispiel 4:

Nachdem die in vitro-Vermehrung von PCV in Zellkulturen gelungen war, wurde die Kapazität von Seren verschiedener Säugetiere, PCV zu neutralisieren, untersucht. Diese Untersuchungen geben einen Hinweis darauf, in welchen Säugetieren sich PCV mit nachfolgender Serokonversion vermehrt bzw. ob Seren Antikörper enthalten, die mit PCV kreuzreagieren. Eine sichere Unterscheidung zwischen diesen beiden Möglichkeiten ist mit der vorhandenen Versuchsanordnung allerdings nicht möglich gewesen.

Verschiedene Seren von Einzeltieren (Hund, Katze, Pferd, Schwein, Affe) bzw. gepoolten Seren mehrerer Tiere (Rind) oder Immunglobulinkonzentrate des Menschen aus gepooltem Plasma wurden im Neutralisationstest - Antikörperverdünnung und konstante Virusmenge (ca. 100 CCID₅₀) - geprüft. Wie die nachfolgende Tabelle 3 zeigt, sind neutralisierende Antikörper gegen porcines Circovirus nur in Schweineseren und humanem Immunglobulin (z.B. Beriglobin7) nachweisbar.

**Tabelle 3:**

| **Nachweis von PCV-neutralisierenden Antikörpern in Seren verschiedener Spezies** | | |
|---|---|---|
| **Spezies** | **Anzahl der untersuchten Seren** | **Anzahl der PCV-positiven Seren** |
| Beriglobin*Chargen | 24 | 24 |
| Hund | 10 | 0 |
| Katze | 10 | 0 |
| Pferd | 10 | 0 |
| Rind | 10 | 0 |
| Schwein | 10 | 10 |
| Affe | 10 | 0 |

| | | |
|---|---|---|
| * gereinigtes humanes Gammaglobulin-Konzentrat aus Spenderpool NT-Index > 0,5 wurde als positiv bewertet | | |

### Beispiel 5:

Die das PCV neutralisierenden sowie nicht-neutralisierenden Antikörper lassen sich auch mit anderen dem Fachmann bekannten in vitro-Methoden, z.B. mit Enzym-Immuno-Assays (EIA) nachweisen. Die Adsorption von PCV an eine feste Phase (z.B. Polystyrol, Nylon oder Cellulose) mit nachfolgender Inkubation der zu untersuchenden Seren und weiterer Inkubation mit gegen die in den Seren enthaltenen primären Antikörper gerichteten enzym-markierten, sekundären Antikörpern führt zur Quantifizierung von gegen PCV gerichteten Antikörpern im zu untersuchenden Serum.

PCV-haltiger Zellkulturüberstand aus PS-Zellen wurde 1:100 in 0,1 M NaOH vorverdünnt und in einer geometrischen Verdünnungsreihe in eine ELISA-Mikrotiterplatte pipettiert (Verdünnungspuffer 0,05 M Na₂CO₃, pH 9,6). Nach dem Fachmann bekannten Methoden des Blockens der Platte, Inkubation mit zu untersuchendem Serum und gegen das zu untersuchende Serum gerichteten markierten Antikörpern wurde die Farbintensität gemessen (Tabelle 4). Das Beispiel zeigt, dass sich Antikörper gegen porcines Circovirus nur in Schweineseren und humanem Immunglobulinkonzentraten nachweisen lassen.

**Tabelle 4:**

| **ELISA von verschiedenen Seren zum Nachweis von Antikörpern gegen porcine Circoviren (Extinktion)** | | | |
|---|---|---|---|
| **Virus-Verdünnung** | **Humanes Immunglobulin-Konzentrat (Beriglobin P)** | **Schweineserum** | **Pferdeserum** |
| 1: 200 | >2,000 | >2,000 | 0,386 |
| 1: 400 | 1,983 | >2,000 | 0,426 |
| 1: 800 | 1,168 | 1,852 | 0,189 |
| 1:1600 | 0,726 | 1,233 | 0,253 |
| 1:3200 | 0,268 | 0,706 | 0,335 |
| 1:6400 | 0,381 | 0,457 | 0,129 |

### Beispiel 6:

Bei der Herstellung von Therapeutika oder Substanzen, die zu deren Herstellung eingesetzt werden sollen, aus biologischem Material müssen potentiell vorhandene Viren inaktiviert oder entfernt werden. Deshalb wurde in einer weiteren Versuchsserie die Thermostabilität von porcinem Circovirus bei 60°C (Pasteurisierung in wässriger Lösung) in verschiedenen Medien getestet:
a) in Eagles minimal essential Medium (EME-Medium)
b) in 5%-iger humaner Serumalbuminlösung (HSA)
c) in Pasteurisierungspuffer für Blutgerinnungs-(FVIII-)Präparate (mit Saccharose und Glycin stabilisierte wässrige Lösung).

Hierfür wurde PCV, wie in Beispiel 2 hergestellt, 1:11 v/v in die drei vorstehend erwähnten Medien gegeben (gespikt) und auf 60°C in Wasserbad erhitzt. Nach den angegebenen Zeiten wurden die Proben für die Titration des verbleibenden Restvirus entnommen und auf PS-Zellen titriert.

Die Titerendablesung erfolgte 7 Tage nach Testansatz mikroskopisch mittels der Beobachtung des cytopathogenen Effekts; die Ergebnisse sind in Tabelle 5 dargestellt.

Wie die Ergebnisse zeigen, ist PCV labil gegen physico-chemische Parameter wie erhöhte Temperatur. Stabilisatoren, die dem Pasteurisierungspuffer von Faktor VIII-Präparaten zugesetzt werden, um diesen Faktor während der Pasteurisierung (Hitzebehandlung bei 60°C in stabilisierter wässriger Lösung) zu stabilisieren, stabilisieren ebenfalls PCV bis zu einem gewissen Grad. Damit können Circoviren zur Untersuchung der Kapazität eines Herstellungsverfahrens oder eines Diagnostikums, Circoviren oder verwandte Viren zu inaktivieren und/oder zu entfernen, verwendet werden.

**Tabelle 5:**

| **Virustiter (log**_{**10**} **CCID**_{**50**}**/ml) nach Inkubation von PCV in verschiedenen Medien bei 60°C** | | | |
|---|---|---|---|
| **Pasteurisierungszeit [h]** | **PCV in Zellkulturmedium** | **PCV in 5% Humanserumalbumin** | **PCV in Faktor VIII-Pasteurisierungspuffer** |
| 0 | 5,9 | 5,8 | 5,8 |
| 1 | ≤1,5 | 1,8 | 4,9 |
| 2 | ≤1,5 | ≤1,5 | 3,5 |
| 4 | ≤1,5 | ≤1,5 | 2,9 |
| 6 | ≤1,5 | ≤1,5 | 1,8 |
| 8 | ≤1,5 | ≤1,5 | ≤1,5 |

### Literaturzusammenstellung:

1. Handa, A. et al., Prevalence of the newly described human circovirus, TTV, in United States blood donors. - Transfusion 40, 245-251 (2000)
2. Tischer, I., Bode, L., Apodaca, J., Timm, H., Peters, D., Rasch, R., Pociuli, S. and Gerike, E. "Presence of antibodies reacting with Porcine Circovirus in sera of humans, mice and cattle"; Arch. Virol. 140, 1427-1439 (1995)
3. Morovsov, I., Sirinarumitr, T., Sorden, S.D., Halbur, P.G., Morgan, M.K., Yoon, K.-I. und Paul, P.S. "Detection of a novel strain of Porcine Circovirus in pigs with postweaning multisystemic wasting syndrom" J. Clin. Microbiol. 36, 2535-2541(1998)
4. Hinrichs, U. et al., Erster Nachweis einer Infektion mit dem porzinen Circovirus Typ 2 in Deutschland. - Tierärztliche Umschau 54, 255-258 (1999)
5. Allan, J.S. "Nonhuman primates as organ donors?" Bull. WHO 77 (1), 62-63 (1999)
6. Kärber, C. "Beitrag zur kollektiven Behandlung pharmakologischer Reihenversuche" Arch. exp. Path. Pharmak. 162:480-487 (1931)
7. Mankertz, A. et al., 1997. Mapping and characterization of the origin of DNA replication of porcine circovirus. J. Gen. Virol. 71: 2562-2566.

## Patentansprüche

1. Verfahren zur Vermehrung von Circoviren, insbesondere porcinen Circoviren (PCV), **dadurch gekennzeichnet, dass** sich aus einer infizierten Zellkultur gewonnene Circoviren nach einmaliger oder mehrmaliger Passage in Zellkulturen des Schweines, des Rindes oder des Menschen in diesen Zellkulturen entwickeln und dabei ein cytopathogener Effekt auftritt.

2. Verfahren zur Neutralisierung oder Entfernung von Circoviren aus biologischem Material, **dadurch gekennzeichnet, dass** es mit einem antikörperhaltigen Substrat wie Schweineserum oder humanem Immunglobulin behandelt oder einem Pasteurisierungsverfahren unterworfen wird.

3. Verfahren zum Nachweis und zur Quantifizierung von gegen Circoviren gerichteten Antikörpern nach dem ELISA-Verfahren, **dadurch gekennzeichnet, dass** Circoviren nach Adsorption an ein Trägermaterial mit dem zu untersuchenden Serum inkubiert, dadurch ein im Serum enthaltener primärer Antikörper gebunden und anschliessend ein gegen den primären Antikörper gerichteter sekundärer, markierter Antikörper damit in Kontakt gebracht wird, wobei dann das von dem gebundenen, markierten Antikörper ausgesendete Signal gemessen wird.

4. Verfahren zum Nachweis und zur Quantifizierung des Circovirus-Antigens nach dem ELISA-Verfahren, **dadurch gekennzeichnet, dass** ein an ein Trägermaterial gebundener Antikörper gegen Circoviren mit dem auf das Circovirus-Antigen zu untersuchenden Serum inkubiert wird, dadurch das Antigen gebunden, dieses mit einem gegen das Antigen gerichteten, markierten Antikörper in Kontakt gebracht und nach dem Auswaschen des nicht gebundenen, markierten Antikörpers das vom gebundenen, markierten Antikörper ausgesendete Signal gemessen wird.

5. Vakzine, **dadurch gekennzeichnet, dass** sie inaktivierte oder avirulente Circoviren enthält.

6. Diagnostikum, **dadurch gekennzeichnet, dass** es inaktivierte oder avirulente Circoviren enthält.

7. Verwendung von Circoviren zur Untersuchung der Kapazität von Arzneimitteln biologischen Ursprungs, von Zusatzstoffen für die Herstellung von Arzneimitteln oder eines Diagnostikums, Circoviren oder verwandte Viren zu inaktivieren und/oder zu entfernen.
